# EUROPEAN PATENT APPLICATION

(11) **EP 4 596 698 A1**
(43) Date of publication of application: **06.08.2025**
(21) Application number: 23871094.1
(22) Date of filing: 28.09.2023
(51) Int. Cl.: C12N 15/85, C12N 15/86

(54) **TARGETING VECTOR, PREPARATION METHOD THEREFOR, AND USE THEREOF**

(30) Priority: 30.09.2022 CN 202211217795
(71) Applicant: Shenzhen Genocury Biotech Co., Ltd., Shenzhen, Guangdong 518122 (CN)
(72) Inventor: JIAN, Zifeng, Shenzhen, Guangdong 518122 (CN)
(74) Representative: Appleyard Lees IP LLP
(86) International application number: PCT/CN2023/122954
(87) International publication number: WO 2024/067870

(57) **Abstract**

Provided are a targeting vector and a method for targeting same to a host cell. The vector comprises a first molecule that binds to an endocytosis receptor of the target cell and a second molecule that promotes the release of a substance carried by the targeting vector into a cytoplasm. When the targeting vector is a viral vector, the first molecule is not part of a viral envelope protein, and the second molecule promotes endosomal escape or lysosomal escape of the targeting vector. The first molecule is designed according to the endocytosis receptor of the cell to be targeted, different types of cells can be targeted, and after a reasonable mutation is carried out on the vector, infection of cells that do not need to be targeted can be avoided, thereby improving the accuracy of the vector.

## Description

### CROSS-REFERENCE TO RELATED APPLICATIONS

The present disclosure claims the benefit of and priority to Chinese Patent Application No. 202211217795.2, entitled "TARGETING VECTOR, PREPARATION METHOD THEREFOR AND USE THEREOF", filed with China National Intellectual Property Administration on September 30, 2022, the entire disclosures of which are incorporated herein by reference in its entirety.

### REFERENCE TO SEQUENCE LISTING SUBMITTED ELECTRONICALLY

The present disclosure includes a sequence listing in XML format submitted electronically, which is incorporated herein by reference in its entirety. The sequence listing was created on September 28, 2023, and entitled as "JYSW-PA-PCT-NO-05-1-seql.xml", with a file size of 1.56 MB.

### TECHNICAL FIELD

The present disclosure belongs to the field of vector delivery, and in particular, relates to a targeting vector and a method for targeting a host cell.

### BACKGROUND

Currently, there are mainly two approaches for drug delivery: (1) by viral vectors, such as adenovirus, adeno-associated virus (AAV), retrovirus and lentivirus, and (2) by non-viral vectors, such as liposome nanoparticles (LNPs), exosomes, virus-like particles (VLP), and antibody-drug conjugates (ADC). Among them, adenovirus, adeno-associated virus, virus-like particles, and antibody-drug conjugates are delivery vectors without liposome envelopes, and retrovirus, lentivirus, nanoliposomes, exosomes, etc., are delivery vectors with liposome envelopes. Viral vectors with envelopes can target and recognize specific receptor proteins on the cell membrane via envelope proteins. For example, vesicular stomatitis virus envelope protein (VSVG) can target and recognize low-density lipoprotein receptor (LDL-R), and baboon endogenous retrovirus (BaEV) envelope glycoprotein can target and recognize ASCT1 receptor and ASCT2 receptor, thus mediating viral vectors to enter target cells expressing the above receptors.

Currently, vectors based on lentiviral framework and VSVG envelope protein have been widely used in clinical treatment, such as in chimeric antigen receptor T-cell therapy (CAR-T), in which CAR-T is prepared by encapsulating a gene of a CAR molecule with lentivirus carrying VSVG and infecting T cells.

Lentiviral vectors are a class of viral vectors engineered from human immunodeficiency virus (HIV) and are a type of retrovirus, the genome of which are RNA, and in which pathogenic genes have been deleted and replaced by exogenous genes of interest. They are pseudotyped viruses which are capable of integrating an exogenous gene into the genome to achieve stable expression through reverse transcriptase and have the characteristics of infecting both dividing and non-dividing cells. Original HIV viruses comprise a complex of gp120 and gp41, which can recognize CD4 molecules, hereby promoting HIV in infecting cells. The engineered lentiviral vectors do not express gp120 and gp41, but express VSVG envelope protein. Since VSVG can target and recognize LDL-R which is widely expressed on a variety of cells, lentiviruses carrying VSVG can widely infect a variety of cells, such as T cells, liver cells, myocardial cells, neurons, endothelial cells, stem cells, and various tumor cells.

The advantages of lentiviral vectors are: (1) long-term expression: lentivirus can integrate an exogenous gene into the genome of a host cell, and facilitate a long-term and stable expression of the gene without loss during cell division and passage, and it is the most favorable candidate for cell experiments and is often used to construct stably transduced strains; (2) high level of safety: they have not yet been found to be pathogenic, and T cells engineered by lentiviral vectors are used in CAR-T cell therapies; and (3) low immunogenicity: direct injection into living tissues does not easily cause an immune response, and it is suitable for animal experiments. The disadvantage thereof is that LDL-R has a broad spectrum of expression on various cells, therefore the specificity thereof is not strong; in addition, there are a variety of cells that are difficult to be infected by lentiviruses, such as quiescent hematopoietic stem cells, quiescent/non-activated T cells, NK cells and B cells.

Currently, the R&D of targeting lentiviruses has already been reported. For example, 1. Targeting lentiviruses with anti-CD3/CD28 antibodies on its surface, which can activate viral particles and infect T cells through expressing anti-CD3 and anti-CD28 antibodies with transmembrane sequences or other antibodies that can activate T cells on the surface of the lentiviruses. This method can effectively promote lentiviral vectors to infect T cells *in vivo.* Although the viruses can be imparted with specificity to a certain extent due to the presence of anti-CD3 antibodies, it can still infect other types of cells due to the presence of wild-type envelope proteins such as VSVG or Cocal (Cocal virus). Therefore, the specificity thereof still needs to be improved. 2. Fusogen-based targeting viruses. Unlike envelope proteins such as VSVG and BaEV, which simultaneously initiates receptor recognition and envelope fusion, the envelope glycoproteins of measles virus (MV) and Nipah virus (NiV) are H/G protein and F protein, wherein H/G protein is responsible for receptor recognition, and after H recognizes a receptor, F protein (Fusogen) undergoes conformational change to mediate the fusion of the viral envelope with the cell membrane. By connecting H/G protein to an antibody, such as anti-CD4 or anti-CD8 antibody, to promote the delivery of the antibody to the extracellular space, the viruses are capable of specifically recognizing CD4+ or CD8+ T cells. Since connecting an antibody to H/G protein increases the steric hindrance of H/G protein, the allosteric F protein may not reach the cell membrane of the target cell, making it difficult to mediate the fusion of the viruses to the envelope.

Besides lentiviruses, AAV vectors are widely used to deliver drugs among gene therapies. Adeno-associated viruses are single-stranded DNA viruses, and it is wildly known in this inudstry that AAV does not cause any human diseases. As vectors for gene therapies, recombinant adeno-associated viruses (rAAV) carry almost the same protein capsid as wild-type AAV. However, the part of the genome encoding viral proteins in the capsid is completely deleted and replaced by therapeutic transgenes. The only part retained of the AAV genome is ITRs, which plays a role in guiding genome replication and viral vector assembly. Currently, most of the clinical trials using AAV focus on four major organs/tissues: eyes, liver, muscles, and central nervous system. Intravenous gene therapies targeting liver have been shown to address metabolic and hematological diseases. Indication for treating Parkinson's disease, hemophilia A, and hemophilia B are most frequently tested in this clinical context.

The advantages of AAV vectors are: (1) not pathogenic, and the vectors are derived from non-pathogenic viruses; (2) the particles are small and can penetrate tissues; and (3) they are easy to be manufactured, and the packaging titer thereof is extremely high. In addition, although AAV vectors of different serotypes have different infection preferences, the specificity thereof is still insufficient.

Other vectors, including LNP and ADC drug delivery systems, have also been widely used in the clinic settings.

LNP-encapsulated messenger ribonucleic acids (mRNAs) have been used for manufacturing COVID-19 vaccines, and a lot of research have been carried out in the field of tumor vaccines. Since the lipid bi-layer has no specificity, it is difficult to target and infect specific cells. By connecting LNP to an anti-CD5 antibody, mRNA encoding CAR-T molecule can be delivered to T cells specifically to produce CAR-T cells. However, LNP in its nature can be easily phagocytized by dendritic cells (DCs), macrophages, etc., and can easily enrich in the liver; in addition, after administration of LNP, a bio-molecular crown formed on the LNP containing hundreds of bio-molecules, such as high-density lipoprotein HDL, will affect the specificity thereof. Therefore, the specificity of LNP is still not sufficient, even if to which an antibody having specificity is connected.

In ADC drugs, an antibody against a cell-specific endocytosis receptor is connected to a drug via a linker that is stable in blood, so that the drug will not be released during intravenous infusion. When the antibody is bound to the cell-specific endocytosis receptor, it is encapsulated by a lysosome, and the linker is degraded by an enzyme in the lysosome, so that the drug can be released. The drug will not be degraded by the lysosome, and it can function without relying on lysosomal escape. ADC drugs exhibit strong specificity. However, based on the ADC delivery methods available nowadays, it is difficult to deliver drugs other than toxins of small molecular compounds, especially bio-macromolecular drugs, as bio-macromolecules such as proteins and nucleic acids can be easily degraded by lysosomes.

In summary, it is generally difficult for the delivery vectors available nowadays to make specific delivery to specific cells, especially specific delivery of bio-macromolecules to specific cells and function properly.

### SUMMARY

In the first aspect of the present disclosure, a targeting vector is provided, comprising a first molecule that binds to an endocytosis receptor of a target cell and a second molecule that promotes the release of a substance loaded on the targeting vector into cytoplasm, when the targeting vector is a viral vector, the first molecule is not part of a viral envelope protein.

The targeting vector involves a variety of forms, which can be enveloped vectors, such as retroviral vectors, lentiviral vectors and lipid nanoparticles, or non-enveloped vectors, such as adenovirus, adeno-associated virus and virus-like particles.

An endocytosis receptor refers to a membrane protein expressed on the cell surface, which can induce endocytosis (or referred to as pinocytosis) after binding with an antibody, a ligand or a specific substance, and an endocytosis receptor such as CD7, CD5, HER2, Mesothelin, etc. Generally, endocytosis receptors comprise domains such as YXXPhi, [D/E]XXXL[L/I] and FXNPXY. In addition, some membrane proteins, such as CD8 molecules, are generally considered to be ineffective in endocytosis due to lack of an endocytosis receptor domain. Therefore, during the screening process of endocytosis domains, CD8 molecules with random sequences chimerically tailed at the intracellular sequence are frequently utilized.

Different cells express different and specific endocytosis receptors. The first molecule can be designed depending on the endocytosis receptor of the cell to be targeted, and the vector can be endocytosed by the target cell via an antibody or ligand (part of the first molecule) that specifically binds to the endocytosis receptor. In this regard, the vector is capable of infecting specific cells without infecting other cells.

After the first molecule binds to the endocytosis receptor, receptor-mediated endocytosis is induced. Receptor-mediated endocytosis is a process in which a cell specifically ingests an extracellular protein or other compounds through a receptor on the cell surface. The receptor on the cell surface, which is highly specific, combines with a corresponding ligand to form a complex, and the plasma membrane involved is then invaginated to form a coated pit, and the pit is detached from the plasma membrane to form a coated vesicle, whereby the extracellular substance is then ingested into the cell. After entering the cell, the coated vesicle sheds its coat and fuses with a small vesicle of the endosome to form a large endosome or an endosome.

The function of the second molecule is to promote endosomal escape or lysosomal escape of the targeting vector after it is endocytosed by the target cell, so as to enter the cytoplasm. After being endocytosed, if the targeting vector fails to make an endosomal escape or lysosomal escape, it will eventually be degraded by the lysosome. Therefore, effective delivery of the substance loaded on the vector into the cytoplasm mandates that the vector is capable of endosomal escape or lysosomal escape.

Common ways for endosomal/lysosomal escape mainly include: (1) destroying endosome/lysosome, e.g., by polyethyleneimine (PEI), which makes the endosome/lysosome expand and break through the "proton sponge" effect, which in turn causes the loaded substance to escape and enter the cytoplasm; (2) reducing the stability of the lysosomal membrane by means of the interaction between the positive charges on the surface of the vector and the negatively charged lysosomal membrane; and (3) enabling the vector to undergo membrane fusion with the endosome/lysosome to make an escape, e.g., by means of modifying the vector to express fusogenic peptide GALA or by enabling the vector to carry a viral envelope protein which is capable of membrane fusion, such as VSVG or variants thereof.

In a specific embodiment, the second molecule is a viral envelope protein and/or a non-viral envelope protein.

In a specific embodiment, the viral envelope protein is at least one of vesicular stomatitis virus envelope glycoprotein VSVG and variants thereof, baboon endogenous retrovirus (BaEV) envelope glycoprotein and variants thereof, feline endogenous retrovirus envelope glycoprotein RD114 and variants thereof, or gibbon ape leukemia virus envelope glycoprotein GALV and variants thereof; and the non-viral envelope protein is at least one of adeno-associated virus (AAV) VP1 and variants thereof, adeno-associated virus (AAV) VP2 and variants thereof, or polyethyleneimine.

Viral envelope proteins, such as VSVG, can promote the fusion of the viral envelope to the endosomal/lysosomal membrane in the endosome/lysosome, thus promoting the release of the substances loaded on the vector. Non-viral envelope proteins, such as AAV VP1 and VP2, can undergo conformational changes under acidic conditions and form perforations on endosomes/lysosomes, thus releasing the substances loaded on the vector. Polyethyleneimine can induce a proton sponge effect in the endosome/lysosome, that is, when the pH in the lysosome decreases, PEI can capture a large number of protons and cause the influx of chloride ions, leading to increased osmotic pressure in the endosome/lysosome, and finally causing the endosome/lysosome to break, thus promoting the release of the substances loaded on the vector.

The variant refers to a mutant that is at least 75% identical to the amino acid sequence of a non-mutant (wild type), and "at least 75% identical to" refers to 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identical to the amino acid sequence of a non-mutant (wild type).

Since the receptor LDL-R of VSVG is widely expressed on activated T cells, hepatocytes, cardiomyocytes, endothelial cells, stem cells and other cells, as well as various tumor cells, pseudotyped lentiviruses carrying VSVG or variants thereof can infect various types of cells. Although VSVG-based pseudotyped viruses possess a broad spectrum of infection ability, there are a variety of cells that do not express or under-express the receptor LDL-R of VSVG, such as quiescent/non-activated NK cells and T cells. In this regard, it is difficult for the VSVG-based pseudotyped viruses to infect these cells. In addition, since LDL-R is widely expressed on a variety of cells, the VSVG-based pseudotyped viruses are lack of specificity. For example, various types of cells express LDL-R, and it is difficult for lentiviruses to accurately transduce only one type of them.

In the present disclosure, as the lentivirus are capable of endosomal/lysosomal escape, it is engineered such that the first molecule is connected to the lentivirus to produce a targeting vector, which can be flexibly applied to different scenes for infecting different cells, and designing the first molecule based on the cells to be infected so that the targeting vector can be applied more broadly with an improvement of its specificity.

In a specific embodiment, the second molecule is selected from the group consisting of VSVG and variants thereof.

In a specific embodiment, the first molecule comprises a transmembrane peptide and an antibody or a ligand that binds to the endocytosis receptor of the target cell. In some embodiments, the first molecule further comprises an extracellular hinge domain. The membrane-expressed protein generally needs a hinge domain, which is beneficial to the extension of the membrane protein, and a CD8 hinge domain is commonly used.

Without affecting the function thereof, the amino acid sequence of the first molecule is not restricted. For example, when the first molecule includes an anti-CD33 antibody, it may be a protein that is at least about 75%, 80%, 85%, 90%, 91%, 92%, 93%, 94%, 95%, 96%, 97%, 98%, 99%, or 99.5% identical to the amino acid sequence of the anti-CD33 antibody.

Different target cells have different endocytosis receptors, and in general, endocytosis receptors include, e.g., HER2, CD20, CD19, CD79A, CD79B, CD56, CD22, CD138, CD37, CD98, CD309, CD33, CD163, CD163B, CD5, CD7, CD169, CD204, CD205, CD209, CD280, CD302, TROP-2, CD19, NECTIN4, 5T4, CD30, FRα, STEAP1, ENPP3, GCC, SLC44A4, NaPi2b, CA9, SC-16, CD142, P-Cadherin, PSMA, ED-B, endothelin receptors ETB, TN-C, Collagen IV, Periostin, CEACAM, c-MET, TDGF1, IGF1R, Mesothelin, TIM1, NCAM1, ZIP6, CD166, GPNMB, SDC1, glycosphingolipid, TfR, Ganglioside, CD74, CLDN18, DPEP3, SLITRK6, PRL-R, LY75, CD48, MUC1, CDKs, B7-H4, STING, KAAG1, CD70, CDH3, LRRC15, EGFR, and ASGPR.

In a specific embodiment, the targeting vector is a lentiviral vector; and the first molecule expressed on the lentiviral vector is a transmembrane protein, and the second molecule expressed by the lentiviral vector is a viral envelope protein which is capable of promoting endosomal escape or lysosomal escape. When the target cell does not express a viral envelope protein receptor, the virus can infect the target cell by means of endocytosis mediated by the first molecule.

In a specific embodiment, the transmembrane protein is selected from the group consisting of an anti-CD7 antibody, an anti-CD19 antibody, an anti-CD33 antibody, an anti-ASGRP antibody or ligand, an anti-mesothelin antibody, and an anti-HER2 antibody; the transmembrane protein may also be a protein that is at least about 75%, 80%, 85%, 90%, 91%, 92%, 93%, 94%, 95%, 96%, 97%, 98%, 99%, or 99.5% identical to the amino acid sequence of at least one of a CD8 signal peptide, a TH-69 heavy chain (VH), a GS linker peptide (linker), a TH-69 light chain (VL), a CD8 hinge domain or a CD8 transmembrane domain of an anti-CD7 antibody; the transmembrane protein may also be a protein that is at least about 75%, 80%, 85%, 90%, 91%, 92%, 93%, 94%, 95%, 96%, 97%, 98%, 99%, or 99.5% identical to the amino acid sequence of at least one of a CD8 signal peptide, a Gemtuzumab light chain (VL), a GS linker peptide (linker), a Gemtuzumab heavy chain (VH), a CD8 hinge domain or a CD8 transmembrane domain of the anti-CD33 antibody; the transmembrane protein may also be a protein that is at least about 75%, 80%, 85%, 90%, 91%, 92%, 93%, 94%, 95%, 96%, 97%, 98%, 99%, or 99.5% identical to the amino acid sequence of at least one of a CD8 signal peptide, a FMC-63 heavy chain (VH), a GS linker peptide (linker), a FMC-63 light chain (VL), a CD8 hinge domain or a CD8 transmembrane domain of the anti-CD19 antibody; the transmembrane protein may also be a protein that is at least about 75%, 80%, 85%, 90%, 91%, 92%, 93%, 94%, 95%, 96%, 97%, 98%, 99%, or 99.5% identical to the amino acid sequence of at least one of a CD8 signal peptide, a ASGPR light chain B11 (VL), a GS linker peptide (linker), a ASGPR heavy chain (VH), a CD8 hinge domain or a CD8 transmembrane domain of the anti-ASGRP antibody; the transmembrane protein may also be a protein that is at least about 75%, 80%, 85%, 90%, 91%, 92%, 93%, 94%, 95%, 96%, 97%, 98%, 99%, or 99.5% identical to the amino acid sequence of at least one of a CD8 signal peptide, a Pertuzumab light chain (VL), a GS linker peptide (linker), a Pertuzumab heavy chain (VH), a CD8 hinge domain or a CD8 transmembrane domain of the anti-HER2 antibody; and the transmembrane protein may also be a protein that is at least about 75%, 80%, 85%, 90%, 91%, 92%, 93%, 94%, 95%, 96%, 97%, 98%, 99%, or 99.5% identical to the amino acid sequence of at least one of a CD8 signal peptide, a PE38 heavy chain (VH), a GS linker peptide (linker), a PE38 light chain (VL), a CD8 hinge domain or a CD8 transmembrane domain of the anti-mesothelin antibody.

In a specific embodiment, a mutation in the viral envelope protein weakens the ability of receptor recognition thereof. For example, the ability of binding to LDL-R is weakened or eliminated, and only the ability of endosomal escape or lysosomal escape is retained, thus completely relying on the specificity of the first molecule. Through the envelope protein with such mutation, the targeting ability of the vector is further improved, so that it can only infect specific target cells.

In a specific embodiment, the first molecule comprises an antibody or ligand, wherein after the antibody binds to the corresponding antigen (endocytosis receptor) on the target cell, endocytosis occurs; and the second molecule comprising mutated VSVG, which jeopardizes the binding ability of VSVG to LDL-R, but at the same time does not affect the ability of lysosomal escape of VSVG, wherein the mutation is insertion, deletion, or substitution. For example, the vesicular stomatitis virus Indiana strain envelope glycoprotein VSVG comprises one of or a combination of the following site mutations: H8 mutation, N9 mutation, Q10 mutation, K47 mutation, K50 mutation, A51 mutation, S183 mutation, S179 mutation, N180 mutation, I182 mutation, M184 mutation, Y209 mutation, I347 mutation, T350 mutation, T352 mutation, E353 mutation, R354 mutation, amino acid deletion at positions 1-18, amino acid deletion at positions 19-36, amino acid deletion at positions 37-51, amino acid deletion at positions 314-384, amino acid deletion at positions 321-374, amino acid deletion at positions 331-364, amino acid deletion at positions 344-354, and amino acid deletion at positions 345-353.

Furthermore, VSVG comprises one of or a combination of the following site mutations: H8 substitution, N9 substitution, Q10 substitution, K47 substitution, K47 deletion, K50 substitution, A51 substitution, S183 substitution, S179 substitution, N180 substitution, I182 substitution, M184 substitution, Y209 substitution, I347 substitution, T350 substitution, T352 substitution, E353 substitution, R354 substitution, amino acid deletion at positions 1-18, amino acid deletion at positions 19-36, amino acid deletion at positions 37-51, amino acid deletion at positions 314-384, amino acid deletion at positions 321-374, amino acid deletion at positions 331-364, amino acid deletion at positions 344-354, and amino acid deletion at positions 345-353.

Furthermore, K47 and/or R354 in the amino acid sequence of VSVG are mutated, for example, the amino acid at position 47 of VSVG is substituted for glutamine Q from lysine K, and/or the amino acid at position R354 is substituted for glutamine Q from arginine R. For example, the amino acid at position 47 of VSVG is deleted.

In a specific embodiment, the viral envelope protein is selected from the group consisting of vesicular stomatitis virus Indiana strain envelope glycoprotein VSVG, Cocal virus envelope glycoprotein, Maraba virus envelope glycoprotein, Morreton virus envelope glycoprotein, Alagoa virus envelope glycoprotein, New Jersey virus envelope glycoprotein, and Carajas virus envelope glycoprotein.

In a specific embodiment, compared with the full region of vesicular stomatitis virus Indiana strain envelope glycoprotein VSVG, Cocal virus envelope glycoprotein, Maraba virus envelope glycoprotein, Morreton virus envelope glycoprotein, Alagoa virus envelope glycoprotein, New Jersey virus envelope glycoprotein, and Carajas virus envelope glycoprotein undergo site mutations at the following corresponding sites: H8 substitution/deletion, N9 substitution/deletion, Q10 substitution/deletion, K47 substitution/deletion, K50 substitution/deletion, A51 substitution/deletion, S183 substitution/deletion, S179 substitution/deletion, N180 substitution/deletion, I182 substitution/deletion, M184 substitution/deletion, Y209 substitution/deletion, I347 substitution/deletion, T350 substitution/deletion, T352 substitution/deletion, E353 substitution/deletion, R354 substitution, amino acid deletion at positions 1-18, amino acid deletion at positions 19-36, amino acid deletion at positions 37-51, amino acid deletion at positions 314-384, amino acid deletion at positions 321-374, amino acid deletion at positions 331-364, amino acid deletion at positions 344-354, and amino acid deletion at positions 345-353.

Amino acid mutations include deletion, substitution and insertion of amino acids. Most studies and experiments focus on substitutions of amino acids, while studies on insertions and deletions of amino acids are still ignored (Savino S., et al., Insertions and deletions in protein evolution and engineering. Biotechnol Adv. (2022)).

The substitution or deletion of an amino acid at the same site may cause unpredictable and completely different effects.

The substitution of the amino acid lysine K at position 47 for glutamine Q (K47Q) and the amino acid arginine R at position 354 for glutamine Q (R354Q) in the extracellular domain of VSV-G can both lead to weakening or even loss of the ability of VSV-G to specifically bind to LDL-R. From the finding that a lentivirus which utilizes a VSV-G comprising K47Q or R354Q mutation in the extracellular domain is used to construct an envelope glycoprotein and the viral envelope of which comprises the above-mentioned first molecule (i.e., transmembrane protein), can still infect a target cell to which the targeting molecule is specific cannot absolutely predict that a lentivirus which utilizes a VSV-G comprising K47 deletion or R354 deletion in the extracellular domain to construct an envelope glycoprotein and the viral envelope of which comprises the above-mentioned first molecule, is still capable of specifically infecting the target cell.

The lentivirus, which utilizes a VSV-G comprising R354 deletion in its extracellular domain to construct an envelope glycoprotein and the viral envelope of which comprises a membrane-expressed anti-CD7 antibody, cannot effectively infect CD7⁺ Jurkat cells; however, the lentivirus, which utilizes a VSV-G comprising K47 deletion in the extracellular domain to construct an envelope glycoprotein and the viral envelope of which comprises a membrane-type anti-CD7 antibody, can effectively infect CD7⁺ Jurkat cells. This further demonstrates that the functional changes in proteins caused by amino acid deletions are difficult to predict.

Taking T cell infection by lentivirus as an example, mutated VSVG and an anti-CD7 antibody are expressed on the envelope of a lentivirus, after the anti-CD7 antibody binds to the CD7 antigen on the surface of a T cell, endocytosis of the lentivirus is mediated, after which the mutated VSVG mediates lysosomal escape, thus completing the targeted infection of the T cell by the lentivirus. Moreover, the lentivirus only infects T cells expressing CD7, while cannot infect cells not expressing CD7.

Substances loaded on the targeting vector are not limited and can be selected according to actual needs, such as small molecular compounds, proteins, polypeptides, RNAs, and DNAs including chimeric antigen receptors CARs and TCRs. The type of CAR is not limited, and the CAR comprises an antigen-binding domain. In some embodiments, the antigen-binding domain is a single-chain variable fragment (scFv) comprising heavy and light chain variable domain that specifically binds to a desired antigen. The scFv is selected from the group consisting of a monoclonal antibody, a chimeric monoclonal antibody, a humanized monoclonal antibody, a human antibody, a nanobody, and a synthetic antibody. In some embodiments, the CAR further comprises a transmembrane domain (e.g., CD8 transmembrane domain) and a signaling domain (e.g., CD3ζ) comprising one or more immunoreceptor tyrosine-based activation motifs (ITAMs). In some embodiments, the CAR comprises one or more co-stimulatory domains. The CAR is not limited by the type of the co-stimulatory domain. In fact, any co-stimulatory domain known in the art can be used, including but not limited to CD28, 4-1BB, DAP10, and DAP12.

Small molecule compounds refer to organic compound molecules with molecular weight that is less than 900 Daltons, especially small molecules that can be used as drugs.

In a specific embodiment, the target cell is lymphocyte, myeloid cell, hematopoietic stem/progenitor cell, or non-hematopoietic cell, especially myeloid cell, hematopoietic stem/progenitor cell, or non-hematopoietic cell, including normal cells and tumor cells.

In a specific embodiment, the endocytosis receptor is the endocytosis receptor of myeloid cell, the endocytosis receptor of hematopoietic stem/progenitor cell, or the endocytosis receptor of non-hematopoietic cell.

In a specific embodiment, the endocytosis receptor is not CD80, TCR, BCR, CD19, CD20, or IL-13Rα.

In a specific embodiment, the endocytosis receptor is not a lymphocyte-specific protein.

The lymphocyte-specific protein refers to a B cell-, T cell- or NK cell-specific protein, and the T cell-specific protein includes CD3, CD28, CD80, 4-1BB, AhR, CD2, CD7, CD4, CD8, CD25, CD44, CD45RA, CD47, CD62L, CD69, CD94, CD95, CD127, CD161, CD183 (CXCR3), CD184 (CXCR4), CD185 (CXCR5), CD193 (CCR3), CD194 (CCR4), CD195 (CCR5), CD196 (CCR6), CD197 (CCR7), CCR10, PD-1, TCRa/b, CD5, CD27, CD45RO, CD45RB, CD57, CD103, CD122, P2RX7, TIGIT, LAG-3, TIM-3, IL6ST, gdTCR (TCRγ, TCRδ), Vdeltal, Vdelta2, NKG2D (KLRK1, CD314), TCR (Va24-Jal8), CD185 (CXCR5), CXCR6, IL-21R, Va7.2, Ja33, CXCR6, IL-18R, KLRB1 (CD161), and VLA4.

The B cell-specific protein includes CD19, CD20, CD21, CD22, CD24, CD38, CD40, CD72, CD32b, CD268, CD269, CD267, CD86, CD80, CD52, CD138, CD27, CD28, CD23, CD84, CD257, CD270, CD37, CD74, and CD269.

The NK cell-specific protein includes CD56, NKp46, CD16, KIR(s), NKG2 protein (NKG2D, KLRK1, CD314), KLRB1 (CD161), KLRDI (CD94), IL2Rb (CD122), IL-21R, SLAMF6 (CD352), SLAMF7 (CD319), and IL-18R.

In another aspect of the present disclosure, a method for preparing the targeting vector is provided, comprising the following steps:
designing a first molecule based on an endocytosis receptor of a target cell,
selecting a second molecule, and
assembling the first molecule, the second molecule and the substance loaded on a vector to prepare the targeting vector.

**In** the first instance, by designing the first molecule, i.e., by modifying the surface of a virus with an antibody/ligand that can target and recognize a specific receptor, and selecting a wild-type viral envelope protein as the second molecule, if the target cell expresses a receptor of the envelope protein of the virus, the presence of the first molecule can promote the infection of the target cell by the targeting vector.

**In** second instance, by designing the first molecule, i.e., by modifying the surface of a virus with an antibody/ligand that can target and recognize a specific receptor, and selecting a wild-type viral envelope as the second molecule, if the target cell under-expresses or does not express a receptor of the envelope protein of the virus, the virus can still infect the target cell through the first molecule.

**In** third instance, by designing the first molecule, i.e., by modifying the surface of a virus with an antibody/ligand that can target and recognize a specific receptor, and selecting a mutated viral envelope protein which has inhibited binding to the receptor but is capable of endosomal/lysosomal escape as the second molecule, regardless of whether the target cell expresses a receptor of the envelope protein of the virus, the virus can infect the target cell in a specific manner through the first molecule, meanwhile, the virus does not infect other cells, thereby greatly improving the targeting accuracy.

Assembly methods for enveloped vector and non-enveloped vector are as follows:
For the assembly of an enveloped vector, taking VSVG as an example: a targeting expression vector can be constructed by mixing a plasmid expressing an antibody/ligand that binds to an endosomal receptor, a lentiviral packaging plasmid such as psPAX2 and pMD.2G (VSVG or VSVG mutants) and a lentiviral expression vector.

For the assembly of a non-enveloped vector, taking AAV as an example: construction of an AAV targeting vector can be realized by linking an antibody/ligand that binds to an endocytosis receptor to the envelope protein of AAV via a transmembrane peptide.

Approaches in which the enveloped vector and the non-enveloped vector enter a cell:
The enveloped vector, such as VSVG-based pseudotyped lentiviral vector, enters a cell by means of binding to **LDL-R** on the surface of the cell membrane via VSVG, and by clathrin-mediated endocytosis. After acidification of an endosome formed by endocytosis, the conformation of the VSVG changes, leading to the fusion of the viral envelope to the endosomal membrane, so that the virus can escape away from the endosome/lysosome and enters the nucleus through a nuclear pore.

As for the non-enveloped vector, the recombinant AAV virus particle enters a cell by means of binding to a glycosylation receptor expressed on the surface of the host cell, and by clathrin-mediated endocytosis. After acidification of an endosome formed after endocytosis, the conformation of some parts of the VP1/VP2 of the viral capsid changes, leading to the virus escaping from the endosome and entering the nucleus through a nuclear pore.

In another aspect of the present disclosure, the targeting vector can be used for the delivery of drugs or vaccines, especially for the delivery of small molecule compounds, proteins, polypeptides, RNAs, or DNAs.

In another aspect of the present disclosure, a method for introducing a substance into a cell is provided, the method comprises bringing the cell into contact with a targeting vector.

In a specific embodiment, the cell is a mammalian cell.

In a specific embodiment, the cell is a normal cell or a cancer cell.

In a specific embodiment, the cell is a T cell, an NK cell, a B cell, a macrophage, a granulocyte, a dendritic cell, a hematopoietic stem cell, a hepatocyte, an islet cell, a nerve cell, and a muscle cell.

In a specific embodiment, the contact is carried out *in vivo* or *in vitro,* for example, it can be administered intravenously, intraperitoneally, intratumorally, intraosseously, or intranodular, so that the targeting vector enters into the subject and comes into contact with the target cell; or the targeting virus is allowed to directly infect the target cell *in vitro.*

The term "mammal" refers to any mammal species such as human, mouse, rat, dog, cat, hamster, guinea pig, rabbit, and livestocks.

The term "viral envelope protein" refers to a naturally occurring viral envelope protein (Envelope Protein), such as VSV-G, BaEV, and RD114. Viral envelope proteins play an important role in virus packaging and host cell infection.

In another aspect of the present disclosure, a composition comprising a targeting vector is provided. The composition can be used as a medication, and the composition can be used for preparing a medication for gene therapy, immunotherapy, cell therapy, treatment of gene defect diseases, treatment of autoimmune diseases, treatment of infectious diseases, or treatment of cancers, wherein the cancers include hematological cancers and solid cancers.

In another aspect of the present disclosure, a method for treating a disease in a subject is provided, the method comprises administering a therapeutically effective amount of a targeting vector or a composition to the subject.

The administration/route of administration of the pharmaceutical composition is conventional in the art, e.g., via oral, nasal, intravenous, intraperitoneal, intracerebral (intracerebral parenchyma), intracerebroventricular, intramuscular, intraocular, intra-arterial, portal vein or intralesional injection, and can also be administered by means of a sustained-release system or an implanted device.

"Treatment" refers to the treatment method described herein which is provided with a subject to achieve at least one of positive therapeutic effects (e.g., a decrease in the number of cancer cells, a decrease in tumor volume, a decrease in the rate of cancer cells infiltrating into peripheral organs, or a decrease in the rate of tumor metastasis or tumor growth). The treatment method for effectively treating a patient may vary depending on a variety of factors, e.g., the disease state, age and body weight of the patient, and the ability of the treatment method to stimulate an anti-cancer response in the subject.

Those skilled in the art will understand that the appropriate dosage level for treatment will vary partly depending on the molecules to be delivered, the indication, the route of administration, and the conditions (body weight, body surface or organ size) and/or state (age and general health condition) of the patient. In some embodiments, clinicians may titrate the dosage and change the route of administration to obtain the best therapeutic effect.

In the present disclosure, a targeting vector is obtained by engineering a vector by combining mechanisms of endocytosis and endosomal/lysosomal escape, such that the vector has a first molecule that can bind to the endocytosis receptor of the target cell. The beneficial effects are as follows:
Based on the specific and efficient endocytosis receptors of different cell types, targeting vectors specific for different cell types can be rapidly developed without blind screening or trial and error verification.

The first molecules designed based on the endocytosis receptor of the cell to be targeted can target different types of cells, especially the cells that do not express or under-express the widely used viral envelope protein receptors (such as LDL-R), and have a wide range of applications.

After reasonable mutations of the vector, the infection of unintended cells can be avoided, and the targeting accuracy is greatly improved.

All publications, documents and patents referred to herein are hereby incorporated by reference in their entirety, as if each individual publication, document or patent is specifically and individually indicated to be incorporated herein by reference in its entirety. In case of conflict, the present disclosure (including any definition herein) shall prevail. However, any references, articles, publications, patents, patent publications and patent applications cited herein are not and should not be regarded as admission or any form of suggestions that they constitute the valid prior art or form part of common sense in any country in the world.

Section headings used herein are for organizational purposes only and are not to be construed as limiting the described subject matter.

### BRIEF DESCRIPTION OF DRAWINGS

FIG. 1 is a schematic diagram of a targeting vector;
FIG. 2 is a schematic diagram of a framework of pGClenti-GFP lentiviral vector;
FIG. 3 is a flow cytometry diagram of peripheral blood NK cells infected by the virus of Example 1;
FIG. 4 is a schematic diagram of VSVG mutants of Example 2;
FIG. 5 is a diagram of detection of capsid protein P24 of 15 groups of lentiviruses in which envelope glycoproteins are constructed with different VSVG mutants;
FIG. 6 is a diagram of titer measurings of 15 groups of lentiviruses in which envelope glycoproteins are constructed with different VSVG mutants;
FIG. 7 is a flow cytometry diagram of CD7⁺ cells infected by the virus of Example 3;
FIG. 8 is a flow cytometry diagram of Jurkat cells, Nalm6 cells and THP1 cells infected by the targeting lentivirus A33-VSV-G-1;
FIG. 9 is a flow cytometry diagram of Jurkat cells and THP1 cells infected by the targeting lentivirus A33-VSV-G-2;
FIG. 10 is a flow cytometry diagram of Jurkat cells and Nalm6 cells infected by the targeting lentivirus A19-VSV-G-2;
FIG. 11 is a diagram of titer measuring of the targeting lentiviruses A19-VSV-G-1 and A19-VSV-G-2;
FIG. 12 is a flow cytometry diagram of Jurkat cells, HuH-7 cells and Hep-G2 cells infected by the targeting lentivirus A-ASGPR-VSV-G-2;
FIG. 13 is a flow cytometry diagram of Jurkat cells and MCF7 cells infected by the targeting lentivirus A-HER-2-VSV-G-2;
FIG. 14 is a flow cytometry diagram of Jurkat cells and Jurkat-MSN cells infected by the targeting lentivirus A-Jurkat-MSN-VSV-G-2;
FIG. 15 is a flow cytometry diagram of Jurkat cells, Nalm6 cells and PBMCs infected by the targeting lentivirus A8-VSV-G-1;
FIG. 16 is a flow cytometry diagram of Jurkat cells and PBMCs infected by the targeting lentivirus A8-VSV-G-2;
FIG. 17 is a flow cytometry diagram of Jurkat cells infected by the lentiviruses dK47-VSV-G-A7, dR354-VSV-G-A7, sK47Q-VSV-G-A7, and sR354Q-VSV-G-A7, respectively; and
FIG. 18 is a flow cytometry diagram of Nalm6 cells infected by the lentiviruses dK47-VSV-G-A7, dR354-VSV-G-A7, sK47Q-VSV-G-A7, and sR354Q-VSV-G-A7, respectively.

### DETAILED DESCRIPTION

### Example 1

### Construction of lentivirus targeting NK using wild-type VSVG

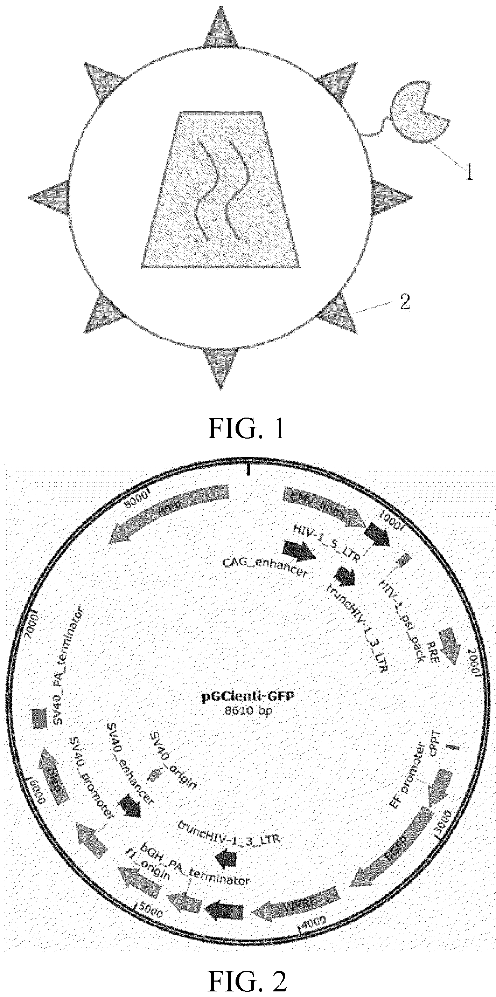

Since NK cells under-expresses LDL-R, the receptor of VSVG, it is difficult for ordinary VSVG lentiviruses to infect NK cells. Therefore, the inventors constructed a membrane-expressed anti-CD7 antibody on the viral envelope of lentivirus, which bound to CD7 on the NK cells and induced endocytosis of lentivirus, followed by VSVG-mediated fusion of viral envelope to endosomal/lysosomal membrane, whereupon lysosomal escape occurred and the GFP loaded on the vector entered into the cells for expression. The structure of the lentiviral targeting vector was shown in FIG. 1, wherein "1" indicates an anti-CD7 antibody as a first molecule that could target endocytosis receptor, and "2" indicates the VSVG as a second molecule that mediated endosomal/lysosomal escape.

### 1. Design of membrane-expressed anti-CD7 antibody

The sequence of the membrane-expressed anti-CD7 antibody comprised, sequentially from 5' to 3' terminus, a CD8 signal peptide, a TH-69 heavy chain (VH), a GS linker peptide (linker), a TH-69 light chain (VL), a CD8 hinge domain, and a CD8 transmembrane domain, wherein the amino acid sequence of the CD8 signal peptide was as set forth in SEQ ID NO: 1:
MALPVTALLLPLALLLHAARP

The amino acid sequence of the TH-69 heavy chain (VH) was as set forth in SEQ ID NO: 2:

EVQLVESGGGLVKPGGSLKLSCAASGLTFSSYAMSWVRQTPEKRLEWVASISSGG
FTYYPDSVKGRFTISRDNARNILYLQMSSLRSEDTAMYYCARDEVRGYLDVWGAGT
TVTVSS

The amino acid sequence of the GS linker peptide (linker) was as set forth in SEQ ID NO: 3:
GGGGSGGGGSGGGGS

The amino acid sequence of the TH-69 light chain (VL) was as set forth in SEQ ID NO: 4:

AAYKDIQMTQTTSSLSASLGDRVTISCSASQGISNYLNWYQQKPDGTVKLLIYYT
SSLHSGVPSRFSGSGSGTDYSLTISNLEPEDIATYYCQQYSKLPYTFGGGTKLEIKR

The amino acid sequence of the CD8 hinge domain was as set forth in SEQ ID NO: 5:
TTTPAPRPPTPAPTIASQPLSLRPEACRPAAGGAVHTRGLDFACD

The amino acid sequence of the CD8 transmembrane domain was as set forth in SEQ ID NO: 6:
IYIWAPLAGTCGVLLLSLVITLYC

### 2. Construction of wild-type VSVG

A wild-type extracellular domain of VSVG comprised an amino acid sequence as set forth in SEQ ID NO: 7:

KFTIVFPHNQKGNWKNVPSNYHYCPSSSDLNWHNDLIGTALQVKMPKSHKAIQ
ADGWMCHASKWVTTCDFRWYGPKYITHSIRSFTPSVEQCKESIEQTKQGTWLNPGFP
PQSCGYATVTDAEAVIVQVTPHHVLVDEYTGEWVDSQFINGKCSNYICPTVHNSTTW
HSDYKVKGLCDSNLISMDITFFSEDGELSSLGKEGTGFRSNYFAYETGGKACKMQYC
KHWGVRLPSGVWFEMADKDLFAAARFPECPEGSSISAPSQTSVDVSLIQDVERILDYS
LCQETWSKIRAGLPISPVDLSYLAPKNPGTGPAFTIINGTLKYFETRYIRVDIAAPILSRM
VGMISGTTTERELWDDWAPYEDVEIGPNGVLRTSSGYKFPLYMIGHGMLDSDLHLSS

KAQVFEHPHIQDAASQLPDDESLFFGDTGLSKNPIELVEGWFSSWKSSIASFFFIIGLIIG
LFLVLRVGIHLCIKLKHTKKRQIYTDIEMNRLGK

### 3. Lentivirus packaging

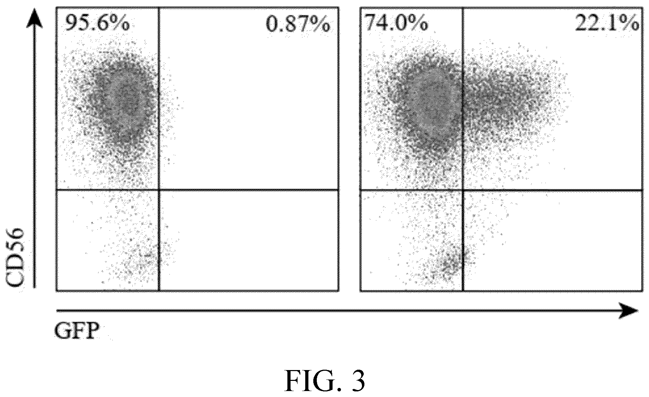

A plasmid carrying the membrane-expressed anti-CD7 antibody, and packaging plasmids of psPAX2 and pMD2.G (wild-type VSVG) were used to package lentivirus expressing pGClenti-GFP (GFP fluorescent protein) (the framework was shown in FIG. 2). In a specific practice, the four plasmids described above were mixed and transduced into 293T cells by PEI, and the supernatant of the medium was collected after 48 hours and centrifuged to obtain lentivirus that could target NK cells. The lentivirus could infect NK cells when it was added to the NK cell culture system. The results were shown in FIG. 3. The left panel was the expression of GFP in the NK cells infected by VSVG lentivirus, and the right panel was the expression of GFP in the NK cells infected by VSVG lentivirus carrying the membrane-expressed anti-CD7 antibody. The ordinary VSVG lentivirus could hardly infect NK cells, while the VSVG lentivirus carrying the membrane-expressed anti-CD7 antibody could effectively target and infect NK target cells, as NK cells did not express LDL-R but highly expressed CD7.

### Example 2

Screening for VSVG mutants without receptor-binding ability but retaining ability of endosomal/lysosomal escape

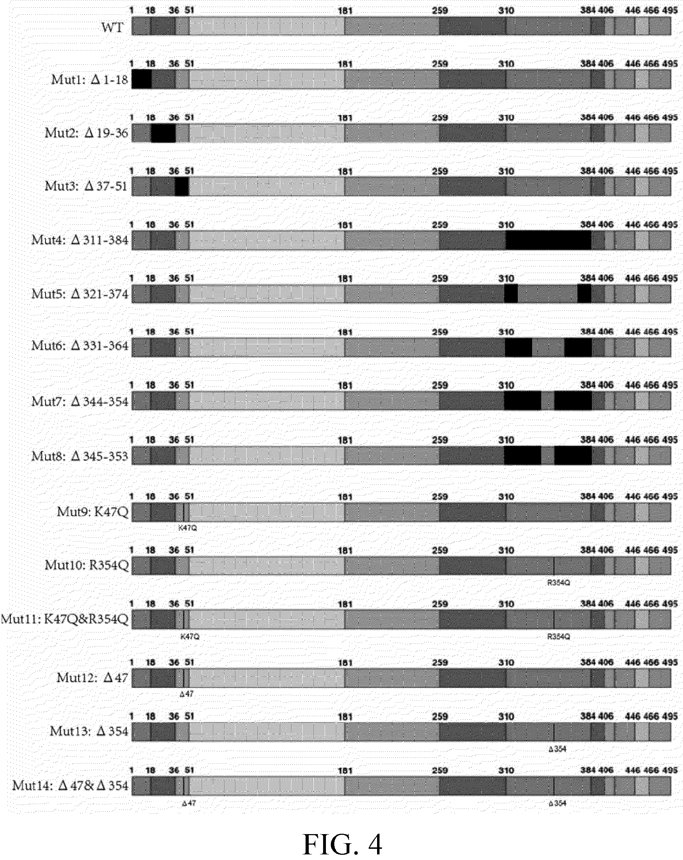

According to the binding site of VSVG to LDL-R, the inventors designed a series of VSVG mutants, as shown in FIG. 4, in which Mut stood for mutant and Δ stood for base deletion, in order to test the binding ability thereof to receptors and the ability of endosomal/lysosomal escape.

15 groups of VSV-G mutants, as shown in FIG. 4, were used to construct viral envelope proteins. Referring to the method for lentivirus packaging described in Example 1, 15 groups of lentiviruses in which the viral envelope comprised the membrane-expressed anti-CD7 antibody were packaged and obtained.

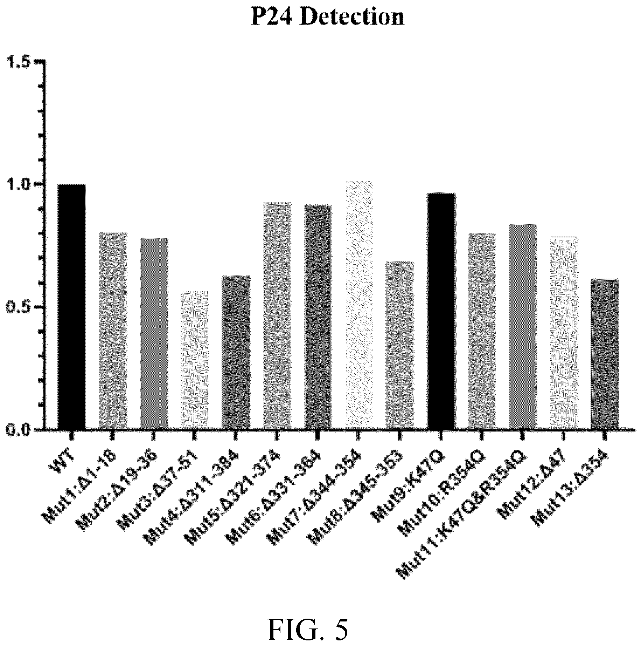

The capsid protein P24 of the 15 groups of packaged lentiviruses was detected, and the results were shown in FIG. 5. The method for detecting capsid protein P24 was well known to those skilled in the art.

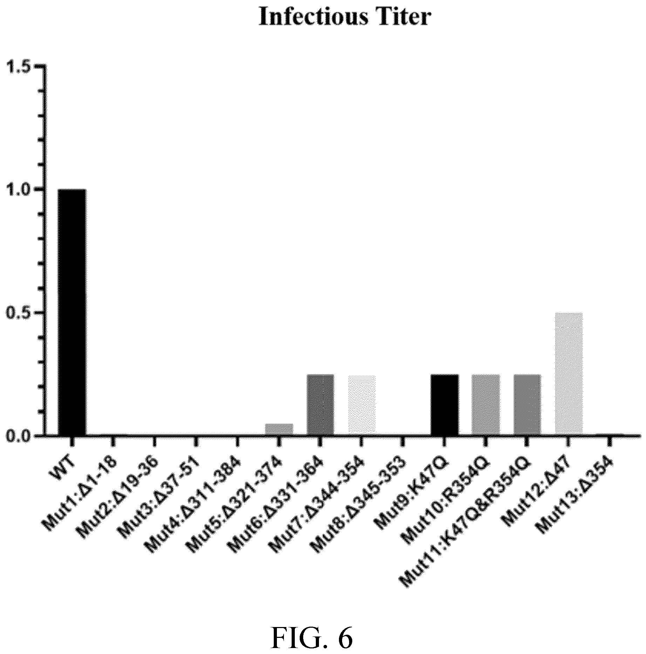

CD7⁺ Jurkat cells were infected by the 15 groups of packaged lentiviruses, respectively, to detect the infectivity of the lentiviruses in each group. The results were shown in FIG. 6.

As could be seen in FIG. 5, lentiviral capsid protein P24 was detected in all the 15 groups of packaged lentiviruses, indicating that lentiviruses were successfully packaged in each group. However, as shown in FIG. 6, the biological activities and titers of the 15 groups of packaged lentiviruses were quite different.

As could be seen in FIG. 6, compared with lentiviruses in which other VSV-G mutants were used to construct envelope glycoproteins, the titer of the lentivirus in which K47-deleted VSV-G mutant was used to construct viral envelope glycoprotein was significantly increased and was approximately twice that of the lentiviruses in which R354Q-mutated VSV-G mutant and K47Q-mutated VSV-G mutant were used to construct viral envelope glycoproteins. Whereas the titer of the lentivirus in which R354-deleted VSV-G mutant was used to construct viral envelope glycoprotein was close to 0, and this lentivirus had almost no infectivity.

### Example 3

### Construction of lentivirus targeting Jurkat cells using mutant VSVG

Mutant VSVG was used, in which the ability of lysosomal escape was retained but the ability of binding to LDL-R was lost, so that it could specifically target cells expressing CD7, and the lentivirus could not infect cells not expressing CD7 but expressing LDL-R.

### 1. Design of anti-CD7 antibody

The same as in Example 1.

### 2. Construction of mutated VSVG

The extracellular domain of the VSVG mutant (in which the amino acid at position 354 was substituted with Q) comprised amino acids as set forth in SEQ ID NO: 8:

KFTIVFPHNQKGNWKNVPSNYHYCPSSSDLNWHNDLIGTALQVKMPKSHKAIQ
ADGWMCHASKWVTTCDFRWYGPKYITHSIRSFTPSVEQCKESIEQTKQGTWLNPGFP
PQSCGYATVTDAEAVIVQVTPHHVLVDEYTGEWVDSQFINGKCSNYICPTVHNSTTW
HSDYKVKGLCDSNLISMDITFFSEDGELSSLGKEGTGFRSNYFAYETGGKACKMQYC
KHWGVRLPSGVWFEMADKDLFAAARFPECPEGSSISAPSQTSVDVSLIQDVERILDYS
LCQETWSKIRAGLPISPVDLSYLAPKNPGTGPAFTIINGTLKYFETRYIRVDIAAPILSRM
VGMISGTTTEQELWDDWAPYEDVEIGPNGVLRTSSGYKFPLYMIGHGMLDSDLHLSS
KAQVFEHPHIQDAASQLPDDESLFFGDTGLSKNPIELVEGWFSSWKSSIASFFFIIGLIIG
LFLVLRVGIHLCIKLKHTKKRQIYTDIEMNRLGK

### 3. Lentivirus packaging

A plasmid carrying the membrane-expressed anti-CD7 antibody, and packaging plasmids of psPAX2 and VSVG mutant were used to package targeting lentivirus expressing pGClenti-GFP (GFP fluorescent protein). In a specific practice, the four plasmids described above were mixed and transduced into 293T cells by PEI, and the supernatant of the medium was collected after 48 hours and centrifuged to obtain lentivirus that could target CD7⁺ cells. The CD7⁺ cells included Jurkat cells, CD7+ NK cells, and CD7+ T cells. The lentivirus could infect the corresponding target cells when added to the culture system of the above cells.

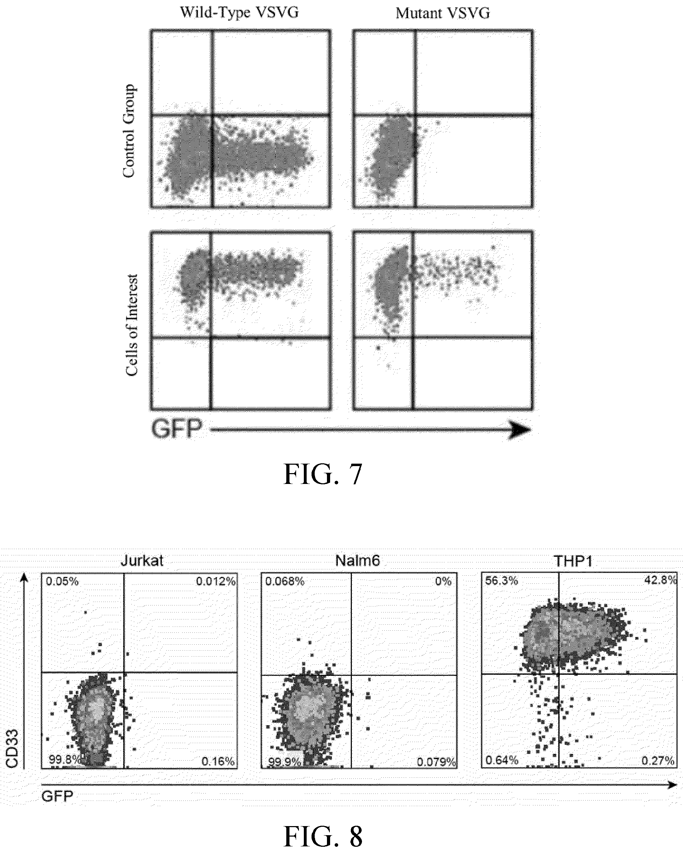

The results were shown in FIG. 7. Raji cells not expressing CD7 and Jurkat cells expressing CD7 were infected by VSVG lentivirus and targeting lentivirus, respectively. The left panels were the results of infection by the non-targeting lentiviral vector, and the right panels were the results of infection by the targeting lentiviral vector. The control group represented Raji cells not expressing CD7, while the experimental group represented CD7+ Jurkat cells. The non-targeting lentiviral vector was capable of infecting both Raji cells and Jurkat cells, whereas the targeting lentiviral vector was only able to infect Jurkat cells rather than Raji cells. In this example, the lentivirus targeting CD7 could not effectively infect Raji cells, but could only effectively infect Jurkat cells, due to the fact that the mutant VSVG used for the targeting lentivirus was not capable of infecting cells, and the specificity thereof depended on the anti-CD7 antibody, which could only target cells expressing CD7. Whereas the wild-type VSVG carried by the non-targeting lentivirus could bind to cells expressing LDL-R, regardless of whether the cells express CD7 or not.

### Example 4

### Construction of lentivirus targeting CD33+ cells

### 1. Design of membrane-expressed anti-CD33 antibody

The sequence of the membrane-expressed anti-CD33 antibody comprised, sequentially from 5' to 3' terminus, a CD8 signal peptide, a Gemtuzumab light chain (VL), a GS linker peptide (linker), a Gemtuzumab heavy chain (VH), a CD8 hinge domain, and a CD8 transmembrane domain.

The amino acid sequence of the Gemtuzumab light chain (VL) was as set forth in SEQ ID NO: 9:

DIQLTQSPSTLSASVGDRVTITCRASESLDNYGIRFLTWFQQKPGKAPKLLMYAAS
NQGSGVPSRFSGSGSGTEFTLTISSLQPDDFATYYCQQTKEVPWSFGQGTKVEVKRTV
AAPSVFIFPPSDEQLKSGTASVVCLLNNFYPREAKVQWKVDNALQSGNSQESVTEQD

SKDSTYSLSSTLTLSKADYEKHKVYACEVTHQGLSSPVTKSFNRGEC

The amino acid sequence of the Gemtuzumab heavy chain (VH) was as set forth in SEQ ID NO: 10:

EVQLVQSGAEVKKPGSSVKVSCKASGYTITDSNIHWVRQAPGQSLEWIGYIYPY
NGGTDYNQKFKNRATLTVDNPTNTAYMELSSLRSEDTAFYYCVNGNPWLAYWGQGT
LVTVSSASTKGPSVFPLAPCSRSTSESTAALGCLVKDYFPEPVTVSWNSGALTSGVHTF
PAVLQSSGLYSLSSVVTVPSSSLGTKTYTCNVDHKPSNTKVDKRVESKYGPPCPPCPAP
EFLGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFF
LYSRLTVDKSRWQEGNVFSCSVMHEALHNHYTQKSLSLSLGK

### 2. Construction of mutant VSV-G according to Example 3

According to Example 3, a mutant VSV-G was constructed, and the extracellular domain of the mutant VSV-G comprised an amino acid sequence as set forth in SEQ ID NO: 8 (in which the amino acid arginine at position 354 was substituted with glutamine Q, mutant VSV-G-1) or an amino acid sequence as set forth in SEQ ID NO: 21 (in which the amino acid lysine K at position 47 was deleted, mutant VSV-G-2).

### 3. Lentivirus packaging

According to Example 3, targeting lentiviruses A33-VSV-G-1 and A33-VSV-G-2 were packaged and respectively used to infect CD33⁺ cells and CD33⁻ cells.

0.5 × 10⁶ Jurkat cells, Nalm6 cells and THP1 cells were respectively prepared and separately re-suspended in 200 µL medium containing 1640 (brand: Elgbio, article number: EH80809) medium and 10% FBS (brand: EXCELL, article number: FSP500). The lentivirus A33-VSV-G-1 targeting CD33⁺ cells was respectively added to each group of the cell culture systems at MOI = 1, mixed uniformly, and placed in an incubator at 37°C for culturing. The expression of GFP in Jurkat cells, Nalm6 cells and THP1 cells was detected on Day 2. The results were shown in FIG. 8.

As could be seen in FIG. 8, the targeting lentivirus A33-VSV-G-1 could not effectively infect Jurkat cells and Nalm6 cells that expressed LDL-R rather than CD33, but could effectively infect THP1 cells expressing CD33.

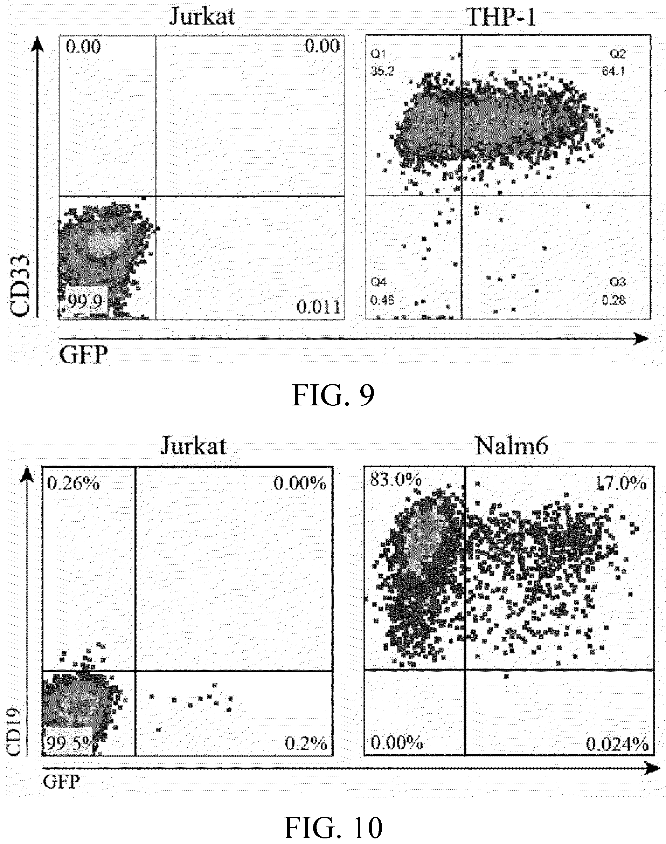

According to the method above for infecting Jurkat cells and THP1 cells by targeting lentivirus A33-VSV-G-1, 1 × 10⁵ Jurkat cells and THP1 cells were respectively prepared and the targeting lentivirus A33-VSV-G-2 was separately added to the Jurkat cell culture system and the THP1 cell culture system at MOI = 1, and the expression of GFP in each culture system was detected on Day 2. The results were shown in FIG. 9.

As could be seen in FIG. 9, the targeting lentivirus A33-VSV-G-2 could not effectively infect Jurkat cells that expressed LDL-R rather than CD33, but could effectively infect THP1 cells expressing CD33.

In this example, the viral envelopes of targeting lentiviruses A33-VSV-G-1 and A33-VSV-G-2, which were constructed with the mutant VSV-G-1 or VSV-G-2, comprised membrane-expressed anti-CD33 antibodies, which could specifically bind to the endocytosis receptor CD33 on the surface of CD33⁺ cells, entered and infected the CD33⁺ cells through endocytosis. Meanwhile, since the mutant VSV-G-1 or VSV-G-2 was used to construct the viral envelope glycoprotein thereof, the ability of the targeting lentiviruses A33-VSV-G-1 and A33-VSV-G-2 to specifically bind to LDL-R was weakened or lost. Therefore, these targeting lentiviruses could not effectively infect cells expressing LDL-R, while the specificity of the targeting lentiviruses A33-VSV-G-1 and A33-VSV-G-2 to infect CD33⁺ cells have been effectively improved.

The flow cytometric antibody used for detecting CD33 by flow cytometry was as follows: product name: APC-CD33, brand: Biolegend, article number: #366606.

### Example 5

### Construction of lentivirus targeting CD19⁺ cells

### 1. Design of membrane-expressed anti-CD19 antibody

The sequence of the membrane-expressed anti-CD19 antibody comprised, sequentially from 5' to 3' terminus, a CD8 signal peptide, an FMC-63 heavy chain (VH), a GS linker peptide (linker), an FMC-63 light chain (VL), a CD8 hinge domain, and a CD8 transmembrane domain.

The amino acid sequence of the FMC-63 heavy chain (VH) was as set forth in SEQ ID NO: 11; and
the amino acid sequence of the FMC-63 light chain (VL) was as set forth in SEQ ID NO: 12.

### 2. Construction of mutant VSV-G according to Example 3

According to Example 3, a mutant VSV-G was constructed, and the extracellular domain of the VSV-G mutant comprised an amino acid sequence as set forth in SEQ ID NO: 8 (in which the amino acid arginine at position 354 was substitutes with glutamine Q, mutant VSV-G-1) or an amino acid sequence as set forth in SEQ ID NO: 21 (in which the amino acid lysine K at position 47 was deleted, mutant VSV-G-2).

### 3. Lentivirus packaging

According to Example 3, the mutant VSV-G-1 and the mutant VSV-G-2 were used to construct targeting lentiviruses A19-VSV-G-1 and A19-VSV-G-2.

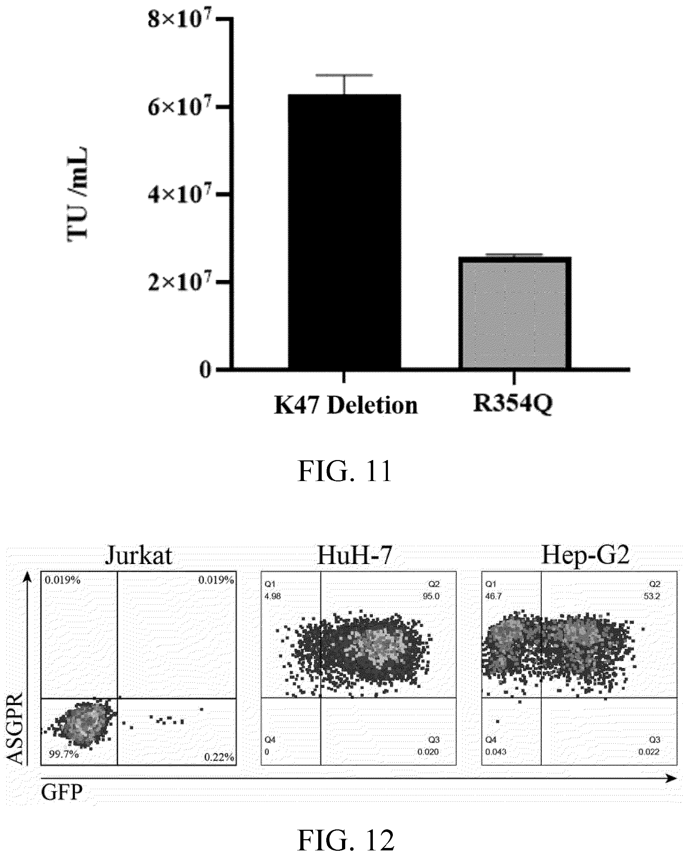

1 × 10⁵ CD19⁻ Jurkat cells and CD19⁺ Nalm6 cells were respectively prepared and separately resuspended in 200 µL medium containing 1640 (brand: Elgbio, article number: EH80809) medium and 10% FBS (brand: EXCELL, article number: FSP500). The targeting lentiviruses A19-VSV-G-1 and A19-VSV-G-2 that targeted CD19⁺ cells were respectively added to each group of cell culture systems at MOI = 1, mixed uniformly, and placed in an incubator at 37°C for culturing. The expression of GFP in Jurkat cells and Nalm6 cells was detected on Day 2. The results of infection of Jurkat cells and Nalm6 cells by the targeting lentivirus A19-VSV-G-2 were shown in FIG. 10; and the results of titer measuring of the targeting lentiviruses A19-VSV-G-1 and A19-VSV-G-2 were shown in FIG. 11.

As could be seen in FIG. 10, the targeting lentivirus A19-VSV-G-2 could not effectively infect Jurkat cells not expressing CD19, but could effectively infect Nalm6 cells expressing CD19.

The targeting lentiviruses A19-VSV-G-1 and A19-VSV-G-2 specifically bound to the endocytosis receptor CD19 on the surface of Nalm6 cells via the anti-CD19 antibody comprised in the viral envelope thereof, and then entered and infected CD19⁺ Nalm6 cells through endocytosis.

As could be seen in FIG. 11, compared with the targeting lentivirus A19-VSV-G-1 in which the mutant VSV-G-1 (R354Q mutation) was used to construct the envelope glycoprotein thereof, the titer of the targeting lentivirus A19-VSV-G-2 in which the mutant VSV-G-2 (K47 deletion) was used to construct the envelope glycoprotein thereof, was significantly increased.

The flow cytometric antibody used for detecting CD19 by flow cytometry was as follows: product name: APC-CD19, brand: SANCI, article number: #S0098.

### Example 6

Construction of lentivirus targeting asialoglycoprotein receptor-positive (ASGPR+) hepatocytes.

N-acetyl galactosamine (GalNAc) or anti-ASGPR antibodies could target asialoglycoprotein receptor (ASGPR). GalNAc could be added to the surface of the lentiviral envelope by modification, or a membrane-expressed anti-ASGPR antibody could be used to prepare lentivirus targeting hepatocytes.

### 1. Design of membrane-expressed anti-ASGPR antibody

The sequence of the membrane-expressed anti-ASGPR antibody comprised, sequentially from 5' to 3' terminus, a CD8 signal peptide, a ASGPR light chain B11 (VL), a GS linker peptide (linker), a ASGPR heavy chain (VH), a CD8 hinge domain, and a CD8 transmembrane domain.

The amino acid sequence of the light chain (VL) of the anti-ASGPR antibody was as set forth in SEQ ID NO: 13:

DIVLTQPPSASGTPGQRVTISCTGSSSGIGNAYVSWYQQLPGKAPKLLIYKNGQRP
SGVSDRFSGSKSGTSASLAISGLRSEDEADYYCAAWDDSLNGWVFGGGTKVTVL

The amino acid sequence of the heavy chain (VH) of the anti-ASGPR antibody was as set forth in SEQ ID NO: 14:

MAEVQLLESGGGLVQPGGSLRLSCAASGFTFSDYYMSWVRQAPGKGLEWVSAI
TTGGGSPNYADSVKGRFTISRDNSKNTLYLQMNSLRAEDTAVYYCARDRTAGYFDY
WGQGALVTVSSGSA

### 2. Construction of mutant VSV-G according to Example 3

According to Example 3, a mutant VSV-G-2 was constructed, wherein the extracellular domain of the mutant VSV-G-2 comprised an amino acid sequence as set forth in SEQ ID NO: 21.

### 3. Lentivirus packaging

According to Example 3, the mutant VSV-G-2 was used to construct targeting lentivirus A-ASGPR-VSV-G-2.

1 × 10⁵ ASGPR⁻ Jurkat cells, ASGPR⁺ HuH-7 cells and ASGPR⁺ Hep-G2 cells were respectively prepared and separately resuspended in 200 µL medium containing DMEM medium and 10% FBS (DMEM, brand: Gibco, article number: #C12430500BT; FBS, brand: EXCELL, article number: #FSP500). The targeting lentivirus A-ASGPR-VSV-G-2 that targeted ASGPR⁺ cells was respectively added to each group of cell culture systems at MOI = 1, mixed uniformly, and placed in an incubator at 37°C for culturing. The expression of GFP in Jurkat cells, HuH-7 cells and Hep-G2 cells was detected on Day 2. The results were shown in FIG. 12.

As could be seen in FIG. 12, the targeting lentivirus A-ASGPR-VSV-G-2 could not effectively infect Jurkat cells not expressing ASGPR, but could effectively infect HuH-7 cells and Hep-G2 cells expressing ASGPR.

The targeting lentivirus A-ASGPR-VSV-G-2 specifically bound to the endocytosis receptor ASGPR on the surface of ASGPR⁺ cells via the anti-ASGPR antibody comprised in the viral envelope thereof, and then entered and infected ASGPR⁺ HuH-7 cells and ASGPR⁺ Hep-G2 cells through endocytosis.

The flow cytometric antibody used for detecting ASGPR by flow cytometry was as follows: product name: PE-ASGPR1, brand: BD, article number: #563655.

### Example 7

### Construction of lentivirus targeting HER2+ cells

### 1. Design of membrane-expressed anti-HER2 antibody

The sequence of the membrane-expressed anti-HER2 antibody comprised, sequentially from 5' to 3' terminus, a CD8 signal peptide, a Pertuzumab light chain (VL), a GS linker peptide (linker), a Pertuzumab heavy chain (VH), a CD8 hinge domain, and a CD8 transmembrane domain.

The amino acid sequence of the Pertuzumab light chain (VL) was as set forth in SEQ ID NO: 15:

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

The amino acid sequence of the Pertuzumab heavy chain (VH) was as set forth in SEQ ID NO: 16:

EVQLVESGGGLVQPGGSLRLSCAASGFTFTDYTMDWVRQAPGKGLEWVADVNP
NSGGSIYNQRFKGRFTLSVDRSKNTLYLQMNSLRAEDTAVYYCARNLGPSFYFDYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV

EVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPG

### 2. Construction of mutant VSV-G according to Example 3

According to Example 3, a mutant VSV-G-2 was constructed, wherein the extracellular domain of the mutant VSV-G-2 comprised an amino acid sequence as set forth in SEQ ID NO: 21.

### 3. Lentivirus packaging

According to Example 3, the mutant VSV-G-2 was used to construct targeting lentivirus A-HER-2-VSV-G-2.

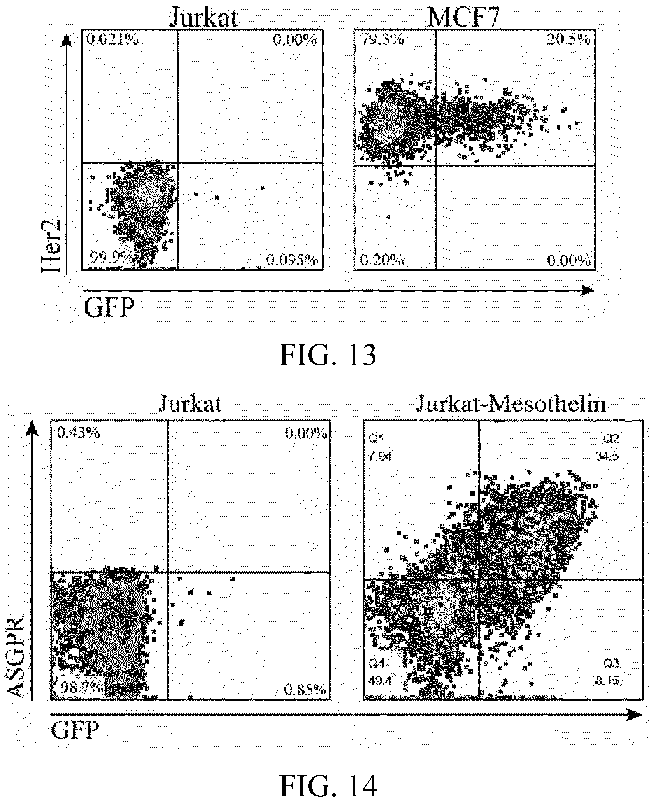

1 × 10⁵ HER-2⁻ Jurkat cells and HER-2⁺ MCF7 cells were respectively prepared and separately resuspended in 200 µL medium containing DMEM medium and 10% FBS (DMEM, brand: Gibco, article number: #C12430500BT; FBS, brand: EXCELL, article number: #FSP500). The targeting lentivirus A-HER-2-VSV-G-2 that targeted HER-2⁺ cells were respectively added to each group of cell culture systems at MOI = 1, mixed uniformly, and placed in an incubator at 37°C for culturing. The expression of GFP in Jurkat cells and MCF7 cells was detected on Day 2. The results were shown in FIG. 13.

As could be seen in FIG. 13, the targeting lentivirus A-HER-2-VSV-G-2 could not effectively infect Jurkat cells not expressing HER-2, but could effectively infect MCF7 cells expressing HER-2.

The targeting lentivirus A-HER-2-VSV-G-2 specifically bound to the endocytosis receptor HER-2 on the surface of MCF7 cells via the anti-HER-2 antibody comprised in the viral envelope thereof, and then entered and infected HER-2⁺ MCF7 cells through endocytosis.

The flow cytometric antibody used for detecting HER-2 by flow cytometry was as follows: product name: APC-CD340 (HER-2), brand: Biolegend, article number: #324407.

### Example 8

### Construction of lentivirus targeting MESOTHELIN+ cells

### 1. Design of membrane-expressed anti-MESOTHELIN antibody

The sequence of the membrane-expressed anti-MESOTHELIN (MSN) antibody comprised, sequentially from 5' to 3' terminus, a CD8 signal peptide, a PE38 heavy chain (VH), a GS linker peptide (linker), a PE38 light chain (VL), a CD8 hinge domain, and a CD8 transmembrane domain.

The amino acid sequence of the PE38 heavy chain (VH) was as set forth in SEQ ID NO: 17:

MQVQLQQSGPELEKPGASVKISCKASGYSFTGYTMNWVKQSHGKSLEWIGLITP
YNGASSYNQKFRGKATLTVDKSSSTAYMDLLSLTSEDSAVYFCARGGYDGRGFDYW
GQGTTVTVSSGV

The amino acid sequence of the PE38 light chain (VL) was as set forth in SEQ ID NO: 18:

DIELTQSPAIMSASPGEKVTMTCSASSSVSYMHWYQQKSGTSPKRWIYDTSKLAS
GVPGRFSGSGSGNSYSLTISSVEAEDDATYYCQQWSGYPLTFGAGTKLEIK

### 2. Construction of mutant VSV-G according to Example 3

According to Example 3, a mutant VSV-G-2 was constructed, wherein the extracellular domain of the mutant VSV-G-2 comprised an amino acid sequence as set forth in SEQ ID NO: 21.

### 3. Lentivirus packaging

According to Example 3, the mutant VSV-G-2 was used to construct targeting lentivirus A-MSN-VSV-G-2.

### 4. Construction of Jurkat-MSN over-expressed cell line

An ordinary lentivirus carrying a nucleic acid encoding MSN, in which the envelope glycoprotein was wild-type VSV-G, was used to infect a Jurkat cell line to construct a Jurkat-MSN cell line over-expressing MSN. The method for constructing the over-expression cell line was well known to those skilled in the art.

1 × 10⁵ MSN⁻ Jurkat cells and MSN⁺ Jurkat-MSN cells were respectively prepared and separately resuspended in 200 µL medium containing 1640 + 10% FBS (1640, brand: Elgbio, article number: #EH80809; FBS, brand: EXCELL, article number: #FSP500). The targeting lentivirus A-MSN-VSV-G-2 that targeted MSN⁺ cells was respectively added to each group of cell culture systems at MOI = 1, mixed uniformly, and placed in an incubator at 37°C for culturing. The expression of GFP in Jurkat cells and Jurkat-MSN cells was detected on Day 2. The results were shown in FIG. 14.

As could be seen in FIG. 14, the targeting lentivirus A-Jurkat-MSN-VSV-G-2 could not effectively infect Jurkat cells not expressing MSN, but could effectively infect Jurkat-MSN cells expressing MSN.

The targeting lentivirus A-MSN-VSV-G-2 specifically bound to the endocytosis receptor MSN on the surface of Jurkat-MSN cells via the anti-MSN antibody ccomprised in the viral envelope thereof, and then entered and infected MSN⁺ Jurkat-MSN cells through endocytosis.

The flow cytometric antibody used for detecting MSN by flow cytometry was as follows: product name: APC-Mesothelin, brand: R&D, article number: #FAB32652A.

### Example 9

Construction of lentivirus targeting CD8+ cells based on non-endocytosis receptor anti-CD8 antibody

### 1. Design of membrane-expressed anti-CD8 antibody

The sequence of the membrane-expressed anti-CD8 antibody comprised, sequentially from 5' to 3' terminus, a CD8 signal peptide, a heavy chain (VH) of anti-CD8 antibody, a GS linker peptide (linker), a light chain (VL) of anti-CD8 antibody, a CD8 hinge domain, and a CD8 transmembrane domain.

The amino acid sequence of the heavy chain (VH) of anti-CD8 antibody was as set forth in SEQ ID NO: 19:

EVQLVESGGGLVQPGGSLRLSCAASGFNIKDTYIHFVRQAPGKGLEWIGRIDPAN
DNTLYASKFQGKATISADTSKNTAYLQMNSLRAEDTAVYYCGRGYGYYVFDHWGQG
TLVTVSS

The amino acid sequence of the light chain (VL) of anti-CD8 antibody was as set forth in SEQ ID NO: 20:

DVQITQSPSSLSASVGDRVTITCRTSRSISQYLAWYQQKPGKVPKLLIYSGSTLQS
GVPSRFSGSGSGTDFTLTISSLQPEDVATYYCQQHNENPLTFGGGTKVEIK

### 2. Construction of mutant VSV-G according to Example 3

According to Example 3, a mutant VSV-G was constructed, and the extracellular domain of the mutant VSV-G comprised an amino acid sequence as set forth in SEQ ID NO: 8 (the amino acid at position 354 was substituted with Q, mutant VSV-G-1) or an amino acid sequence as set forth in SEQ ID NO: 21 (the amino acid lysine K at position 47 was deleted, mutant VSV-G-2).

### 3. Lentivirus packaging

According to Example 3, the mutant VSV-G-1 or the mutant VSV-G-2 was used to package targeting lentivirus A8-VSV-G-1 or A8-VSV-G-2, the viral envelope of which comprised the membrane-expressed anti-CD8 antibody.

1 × 10⁵ Jurkat cells, Nalm6 cells and PBMCs were respectively prepared and separately resuspended in 200 µL medium. The medium for resuspending Jurkat cells and Nalm6 cells contained 1640 (brand: Elgbio, article number: EH80809) medium and 10% FBS (brand: EXCELL, article number: FSP500); and the medium for resuspending PBMCs comprised XVT medium (product name: PRIME-XV T cell CDM, brand: IRVINE (FUJIFILM), article number: 91154), IL-7 with a final concentration of 20 ng/mL (product name: IL-7 Protein, Human, Recombinant, brand: Sino Biological, article number: 11821-HNAE), and IL-15 with a final concentration of 20 ng/mL (product name: IL-15 Protein, Human, Recombinant (His Tag), brand: Sino Biological, article number: 10360-H07E).

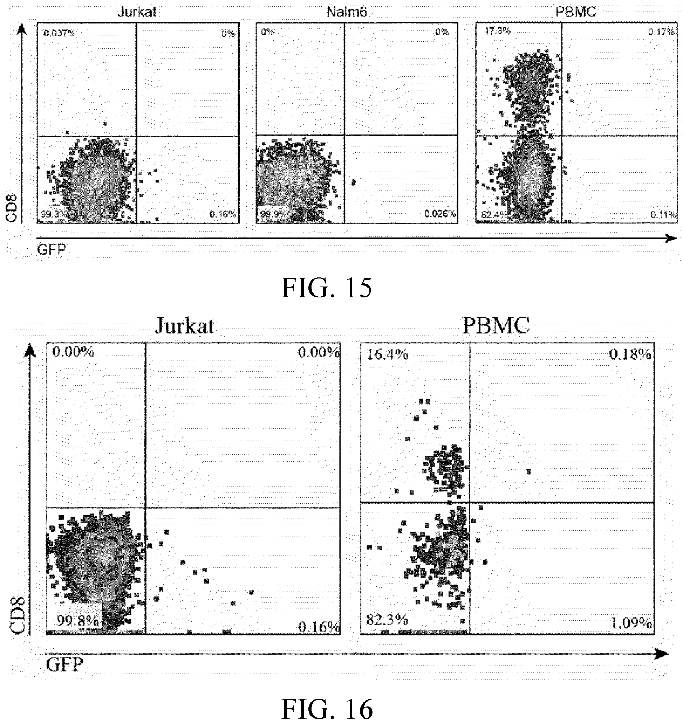

The targeting lentivirus A8-VSV-G-1 was respectively added to Jukart cell, Nalm6 cell and PBMC cell culture systems at MOI = 1, mixed uniformly, placed in an incubator at 37°C. The expression of GFP in Jurkat cells, Nalm6 cells and PBMCs was detected on Day 2. The results were shown in FIG. 15.

As could be seen in FIG. 15, the targeting lentivirus A8-VSV-G-1 constructed with mutant VSV-G-1 could neither effectively infect Jurkat cells and Nalm6 cells that expressed LDL-R but not CD8, nor effectively infect PBMCs expressing CD8.

1 × 10⁵ Jurkat cells and PBMCs were taken respectively. According to the method above for uniformly mixing the targeting lentivirus A8-VSV-G-1 with each group of cells and culturing the mixture, the targeting lentivirus A8-VSV-G-2 was respectively added to the Jurkat cell culture system and the PBMC culture system at MOI = 1. The expression of GFP in Jurkat cells and PBMCs was detected on Day 2. The results were shown in FIG. 16.

As could be seen in FIG. 16, the targeting lentivirus A8-VSV-G-2 could neither effectively infect Jurkat cells that expressed LDL-R but not CD8, nor effectively infect PBMCs expressing CD8.

After the CD8⁺ cells were brought into contact with the targeting lentivirus A8-VSV-G-1 or A8-VSV-G-2, the membrane-expressed anti-CD8 antibody comprised in the viral envelope of the targeting lentivirus A8-VSV-G-1 or A8-VSV-G-2 specifically bound to the receptor CD8 on the surface of CD8⁺ cells in PBMCs. However, since CD8 was a non-endocytosis receptor, it could not efficiently mediate endocytosis, the targeting lentivirus A8-VSV-G-1 or A8-VSV-G-2 could not effectively enter and infect CD8+ cells.

From the results, it was found that after CD8+ cells were brought into contact with the lentivirus, although the CD8 receptor could bind to the antibody, no effective endocytosis would occur, and the lentiviral vector based on the membrane-expressed anti-CD8 antibody could not effectively mediate the infection of CD8+ cells. Therefore, a receptor lacking the ability of efficient endocytosis was not suitable to be the first molecule for constructing the targeting vector of the present disclosure.

The flow cytometric antibody used for detecting CD8 by flow cytometry was as follows: APC-CD8, brand: BD, article number: 566852.

### Example 10

Comparison of infection efficiency of lentivirus with K47 deletion or R354 deletion in the extracellular domain of VSV-G.

An envelope plasmid carrying a nucleic acid encoding the membrane-expressed anti-CD7 antibody in Example 1 and a nucleic acid encoding VSV-G in which the extracellular domain comprised K47 deletion (K47 deletion-A7 envelope plasmid), pMDLg/pRRE packaging plasmid, pRSV-REV packaging plasmid, and lentiviral GFP plasmid were prepared, wherein the K47 deletion-A7 envelope plasmid was synthesized by a conventional molecular cloning method.

According to the packaging method for packaging and preparing lentivirus in Example 3, lentivirus dK47-VSV-G-A7 was packaged and prepared.

Among the four plasmids described above, the envelope plasmid in which the extracellular domain of VSV-G comprised K47 deletion was replaced by an envelope plasmid in which the extracellular domain of VSV-G comprised R354 deletion, K47Q mutation or R354Q mutation. According to the method above for packaging lentiviral vector dK47-VSV-G-A7, lentivirus dR354-VSV-G-A7, sK47Q-VSV-G-A7 or sR354Q-VSV-G-A7 in which the extracellular domain of VSV-G comprised R354 deletion, K47Q mutation or R354Q mutation, respectively, was packaged and prepared.

The extracellular domain of VSV-G comprising K47 deletion comprised an amino acid sequence as set forth in SEQ ID NO: 21;
the extracellular domain of VSV-G comprising R354 deletion comprised an amino acid sequence as set forth in SEQ ID NO: 22;
the extracellular domain of VSV-G comprising K47Q mutation comprised an amino acid sequence as set forth in SEQ ID NO: 23; and
the extracellular domain of VSV-G comprising R354Q mutation comprised an amino acid sequence as set forth in SEQ ID NO: 8.

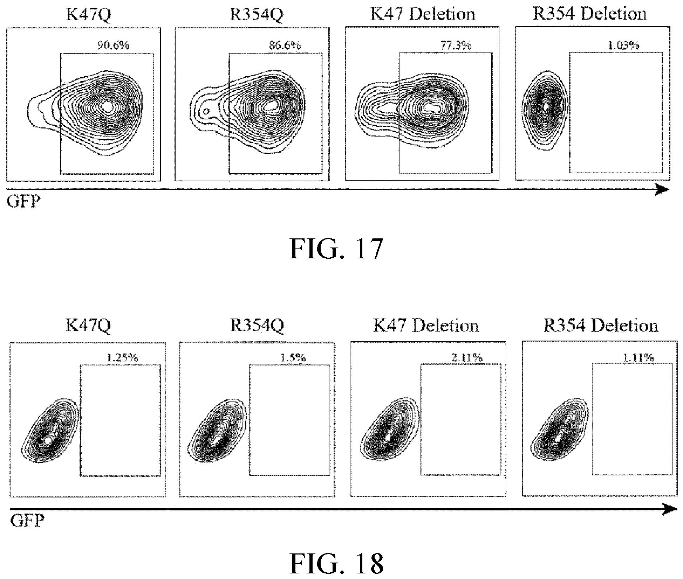

The above four types of lentiviruses were respectively added to four groups of CD7⁺ Jurkat cells at MOI = 1, mixed uniformly, and infected at room temperature for 10 minutes, then 10 mL of DPBS buffer was added, mixed uniformly and then centrifuged at 500 g for 3 minutes. The supernatant was removed and discarded, and 1 mL of 1640 medium containing 10% FBS (brand: ELGBIO, article number: #EH80809; FBS serum: brand: EXCELL, article number: #FSP500) was added to respectively resuspend the four groups of CD7⁺ Jurkat cells, for culture *in vitro* in an incubator at 37°C and 5% CO₂ concentration. The expression of GFP was detected on Day2. The results were shown in FIG. 17.

As could be seen in FIG. 17, the lentiviruses dK47-VSV-G-A7, sK47Q-VSV-G-A7 and sR354Q-VSV-G-A7, in which the extracellular domains of VSV-G comprised K47 deletion, R354Q mutation and K47Q mutation, respectively, could all effectively infect CD7⁺ Jurkat cells by specifically binding to the endocytosis receptor CD7 expressed on Jurkat cells, followed by endocytosis. Whereas it was difficult for the lentivirus dR354-VSV-G-A7, in which the extracellular domain of VSV-G comprised R354 deletion, to infect CD7⁺ Jurkat cells by endocytosis. It indicated that the presence of R354 deletion in the extracellular domain of VSV-G made unpredictable and unknown changes, which are different from that with K47 deletion, regarding the function of VSV-G. Even though the viral envelope thereof comprised the membrane-expressed anti-CD7 antibody, the ability of infecting CD7⁺ cells of the lentivirus dR354-VSV-G-A7 was still lost.

According to the methods for virus infection and *in vitro* culture described above, the above four types of lentiviruses dK47-VSV-G-A7, sK47Q-VSV-G-A7, sR354Q-VSV-G-A7 and dR354-VSV-G-A7 were respectively added to four groups of CD7⁻ Nalm6 cells at MOI = 1, for cell culture *in vitro* in an incubator at 37°C and 5% CO₂ concentration. The expression of GFP was detected on Day 2. The results were shown in FIG. 18.

As could be seen in FIG. 18, none of the lentiviruses dK47-VSV-G-A7, dR354-VSV-G-A7, sK47Q-VSV-G-A7 and sR354Q-VSV-G-A7 could infect CD7⁻ Nalm6 cells, which indicated that the K47 deletion comprised in the extracellular domain of VSV-G did not affect the specificity of the lentivirus dK47-VSV-G-A7 to infect CD7⁺ cells.

The above description only refers to particular embodiments of the present disclosure. However, the scope of protection of the present disclosure is not limited thereto. Any changes or substitutions that are easily conceivable to those skilled in the technical field within the technical scope disclosed by the present disclosure should be included in the scope of protection of the present disclosure. Therefore, with regard to the scope of protection of the present disclosure, the scope of protection of the claims shall prevail.

## Claims

1. A targeting vector comprising a first molecule that binds to an endocytosis receptor of a target cell and a second molecule that promotes the release of a substance loaded on the targeting vector into cytoplasm, when the targeting vector is a viral vector, the first molecule is not part of a viral envelope protein.

2. The targeting vector according to claim 1, wherein the second molecule promotes the targeting vector to undergo endosomal escape or lysosomal escape.

3. The targeting vector according to claim 1, wherein the targeting vector is an enveloped vector or a non-enveloped vector.

4. The targeting vector according to claim 3, wherein the enveloped vector is selected from the group consisting of retroviral vector, lentiviral vector, and lipid nanoparticles; and the non-enveloped vector is selected from the group consisting of adenovirus, adeno-associated virus, and virus-like particles.

5. The targeting vector according to claim 1, wherein the second molecule is a viral envelope protein and/or a non-viral envelope protein.

6. The targeting vector according to claim 5, wherein the viral envelope protein is at least one of vesicular stomatitis virus envelope glycoprotein VSVG and variants thereof, Cocal virus envelope glycoprotein and variants thereof, Maraba virus envelope glycoprotein and variants thereof, Morreton virus envelope glycoprotein and variants thereof, Alagoa virus envelope glycoprotein and variants thereof, New Jersey virus envelope glycoprotein and variants thereof, Carajas virus envelope glycoprotein and variants thereof, baboon endogenous retrovirus (BaEV) envelope glycoprotein and variants thereof, feline endogenous retrovirus envelope glycoprotein RD114 and variants thereof, or gibbon ape leukemia virus envelope glycoprotein GALV and variants thereof; and the non-viral envelope protein is at least one of adeno-associated virus (AAV) VP1 and variants thereof, adeno-associated virus (AAV) VP2 and variants thereof, or polyethyleneimine.

7. The targeting vector according to claim 5, wherein the second molecule is selected from the group consisting of VSVG and variants thereof, and Cocal virus envelope glycoprotein and variants thereof.

8. The targeting vector according to claim 1, wherein the first molecule comprises a transmembrane peptide and an antibody or a ligand that binds to the endocytosis receptor of the target cell.

9. The targeting vector according to claim 8, wherein the first molecule further comprises an extracellular hinge domain.

10. The targeting vector according to claim 1, wherein the endocytosis receptor is selected from the group consisting of: HER2, CD20, CD19, CD79A, CD79B, CD56, CD22, CD138, CD37, CD98, CD309, CD33, CD163, CD163B, CD5, CD7, CD169, CD204, CD205, CD209, CD280, CD302, TROP-2, CD19, NECTIN4, 5T4, CD30, FRα, STEAP1, ENPP3, GCC, SLC44A4, NaPi2b, CA9, SC-16, CD142, P-Cadherin, PSMA, ED-B, endothelin receptors ETB, TN-C, Collagen IV, Periostin, CEACAM, c-MET, TDGF1, IGF1R, Mesothelin, TIM1, NCAM1, ZIP6, CD166, GPNMB, SDC1, glycosphingolipid, TfR, Ganglioside, CD74, CLDN18, DPEP3, SLITRK6, PRL-R, LY75, CD48, MUC1, CDKs, B7-H4, STING, KAAG1, CD70, CDH3, LRRC15, EGFR, and ASGPR.

11. The targeting vector according to claim 1, wherein the targeting vector is a lentiviral vector; and the first molecule expressed by the lentiviral vector is a transmembrane protein, and the second molecule expressed by the lentiviral vector is a viral envelope protein.

12. The targeting vector according to claim 11, wherein the viral envelope protein is mutated such that the ability of receptor recognition thereof is weakened.

13. The targeting vector according to claim 12, wherein the mutation is deletion or substitution.

14. The targeting vector according to claim 12 or 13, wherein the viral envelope protein is selected from the group consisting of vesicular stomatitis virus Indiana strain envelope glycoprotein VSVG, Cocal virus envelope glycoprotein, Maraba virus envelope glycoprotein, Morreton virus envelope glycoprotein, Alagoa virus envelope glycoprotein, New Jersey virus envelope glycoprotein, and Carajas virus envelope glycoprotein.

15. The targeting vector according to claim 14, wherein the vesicular stomatitis virus Indiana strain envelope glycoprotein VSVG comprises one of or a combination of the following site mutations: H8 substitution/deletion, N9 substitution/deletion, Q10 substitution/deletion, K47 substitution/deletion, K50 substitution/deletion, A51 substitution/deletion, S183 substitution/deletion, S179 substitution/deletion, N180 substitution/deletion, I182 substitution/deletion, M184 substitution/deletion, Y209 substitution/deletion, I347 substitution/deletion, T350 substitution/deletion, T352 substitution/deletion, E353 substitution/deletion, R354 substitution, amino acid deletion at positions 1-18, amino acid deletion at positions 19-36, amino acid deletion at positions 37-51, amino acid deletion at positions 314-384, amino acid deletion at positions 321-374, amino acid deletion at positions 331-364, amino acid deletion at positions 344-354, and amino acid deletion at positions 345-353.

16. The targeting vector according to claim 14, wherein compared with the full region of the vesicular stomatitis virus Indiana strain envelope glycoprotein VSVG, Cocal virus envelope glycoprotein, Maraba virus envelope glycoprotein, Morreton virus envelope glycoprotein, Alagoa virus envelope glycoprotein, New Jersey virus envelope glycoprotein, and Carajas virus envelope glycoprotein undergo site mutations at the following corresponding sites: H8 substitution/deletion, N9 substitution/deletion, Q10 substitution/deletion, K47 substitution/deletion, K50 substitution/deletion, A51 substitution/deletion, S183 substitution/deletion, S179 substitution/deletion, N180 substitution/deletion, I182 substitution/deletion, M184 substitution/deletion, Y209 substitution/deletion, I347 substitution/deletion, T350 substitution/deletion, T352 substitution/deletion, E353 substitution/deletion, R354 substitution, amino acid deletion at positions 1-18, amino acid deletion at positions 19-36, amino acid deletion at positions 37-51, amino acid deletion at positions 314-384, amino acid deletion at positions 321-374, amino acid deletion at positions 331-364, amino acid deletion at positions 344-354, and amino acid deletion at positions 345-353.

17. The targeting vector according to claim 11, wherein the transmembrane protein comprises an anti-CD7 antibody, an anti-CD19 antibody, an anti-CD33 antibody, an anti-ASGRP antibody or ligand, an anti-mesothelin antibody, and an anti-HER2 antibody.

18. The targeting vector according to claim 1, wherein the substance is at least one of a small molecule compound, a protein, a polypeptide, an RNA, or a DNA.

19. The targeting vector according to claim 1, wherein the target cell is a lymphocyte, a myeloid cell, a hematopoietic stem/progenitor cell, or a non-hematopoietic cell.

20. The targeting vector according to claim 1, wherein the endocytosis receptor is not a lymphocyte-specific protein.

21. A method for manufacturing the targeting vector according to any one of claims 1 to 20, comprising the following steps:
designing a first molecule according to an endocytosis receptor of a target cell,
selecting a second molecule, and
assembling the first molecule and the second molecule with a substance loaded on a vector to manufacture the targeting vector.

22. Use of the targeting vector according to any one of claims 1 to 20 in the delivery of a drug or a vaccine.

23. The targeting vector according to any one of claims 1 to 20, for use in the delivery of a small molecule compound, a protein, a polypeptide, an RNA, or a DNA.

24. A method for introducing a substance into a cell, the method comprises bringing the cell into contact with the targeting vector according to any one of claims 1 to 20.

25. The method according to claim 24, wherein the cell is a mammalian cell.

26. The method according to claim 24, wherein the cell is a normal cell or a cancer cell.

27. The method according to claim 24, wherein the cell is a T cell, an NK cell, a B cell, a macrophage, a granulocyte, a dendritic cell, a hematopoietic stem cell, a hepatocyte, an islet cell, a nerve cell, or a muscle cell.

28. The method according to claim 24, wherein the contact is carried out *in vivo* or *in vitro.*

29. A composition comprising the targeting vector according to any one of claims 1 to 20.

30. The composition according to claim 29, for use in a medicament.

31. The composition according to claim 30, for use in gene therapy, immunotherapy, cell therapy, treatment of gene defect diseases, treatment of autoimmune diseases, treatment of infectious diseases, and treatment of cancers, wherein the cancers comprise hematological cancers and solid cancers.

32. A method for treating a disease in a subject, wherein a therapeutically effective amount of the targeting vector according to any one of claims 1 to 20 or the composition according to any one of claims 29 to 31, is administered to the subject.
